# EUROPEAN PATENT APPLICATION

(11) **EP 4 603 020 A1**
(43) Date of publication of application: **20.08.2025**
(21) Application number: 24158042.2
(22) Date of filing: 16.02.2024
(51) Int. Cl.: A61B 5/273, A61B 5/00, H01R 13/62

(54) **MAGNET-LOADED TRANSCEIVER ASSEMBLY FOR RECEIVING AND TRANSMITTING SIGNALS FROM AND TO A BODY TISSUE**

(71) Applicant: ETH Zurich, 8092 Zurich (CH)
(72) Inventor: LAMBERCY, Olivier, 8044 Zürich (CH); GASSERT, Roger, 6512 Giubiasco (CH); WYSER, Dominik, 8305 Dietlikon (CH); SOLER, Flurin, 8006 Zürich (CH)

(57) **Abstract**

A transceiver assembly (1) for receiving signals of a body tissue (2) and/or transmitting signals to a body tissue (2) comprises at least one holding device (3) and at least one transceiver device (4). The transceiver device (4) comprises at least one first transceiver-magnetic element (5) and the holding device (3) comprises at least one first holding-magnetic element (6), the first transceiver-magnetic element (5) and the first holding-magnetic element (6) being arranged after one another with respect to a connection direction (C) and being configured to exert a magnetic force to one another. The transceiver device (4) comprises at least one second transceiver-magnetic element (7) and the holding device (3) comprises at least one second holding-magnetic element (8) being arranged after one another with respect to a transverse direction (T) and are configured to exert a magnetic force to one another. The transceiver device (4) is connectable to the holding device (3) via an overall magnetic force exerted by the first and second transceiver-magnetic elements (5; 7) and by the first and second holding-magnetic elements (6; 8).

## Description

### TECHNICAL FIELD

The present invention relates to a transceiver assembly according to claim 1 and to a method of manufacturing a transceiver assembly according to claim 15.

### PRIOR ART

There is a growing interest in reliably and non-invasively transmitting and receiving signals such as biosignals from a body tissue. Of particular interest are human brain activity measurements for diagnostic, monitoring and treatment purposes, as well as input signals for software or hardware systems. This is typically driven by the increasing availability of wearable bio-transceivers and brain imaging systems fueled by recent technological advances and miniaturization. These systems make non-invasive brain imaging accessible to a wider population, enabling exciting opportunities for better understanding the brain, diagnosing neurological disorders and developing treatments, as well as interfacing with brain health and gaming apps.

The immanent importance of usability for clinical applications was repeatedly highlighted by medical healthcare workers. Therefore, a crucial aspect for the success and applicability of wearable brain imaging is the simple, flexible and reliable placement of transceivers, ensuring high usability. Achieving optimal signal quality without causing discomfort requires finding the right balance of contact pressure for optimal signal quality and wearing comfort. This balance also influences the robustness of the system during head movements in wearable applications.

Furthermore, typical measurement locations for functional near-infrared spectroscopy (fNIRS) or electroencephalography (EEG) include areas with hair, such as sensorimotor or occipital brain regions. Therefore, a transceiver assembly is required to adapt to users with varying hair characteristics, ranging from bald to users with a substantial amount of hair.

### SUMMARY OF THE INVENTION

It is an object of the present invention to provide a transceiver assembly that is flexible, yet comfortable and easy-to use.

This object is achieved with a transceiver assembly according to claim 1. In particular, a transceiver assembly for receiving signals of a body tissue and/or transmitting signals to a body tissue is provided. The transceiver assembly comprises at least one holding device and at least one transceiver device. The transceiver device is configured to receive a signal from a body tissue and/or to transmit a signal to a body tissue. The transceiver device is connectable to the holding device along a connection direction. The transceiver device comprises at least one first transceiver-magnetic element and the holding device comprises at least one first holding-magnetic element. The first transceiver-magnetic element and the first holding-magnetic element are arranged after one another with respect to the connection direction and are configured to exert a magnetic force to one another. The transceiver device comprises at least one second transceiver-magnetic element and the holding device comprises at least one second holding-magnetic element. The second transceiver-magnetic element and the second holding-magnetic element are arranged after one another with respect to a transverse direction running perpendicularly to the connection direction and are configured to exert a magnetic force to one another. The transceiver device is connectable to the holding device via an overall magnetic force exerted by the first and second transceiver-magnetic elements and by the first and second holding-magnetic elements.

That is, the transceiver device is preferably connectable to the holding device via an overall magnetic force exerted by the first and second transceiver-magnetic elements and by the first and second holding-magnetic elements. In the thus formed connected state of the transceiver device with the holding device, the transceiver device is connected to the holding device by the first and second transceiver-magnetic elements and by the first and second holding-magnetic elements.

This arrangement of the first transceiver-magnetic element and the first holding-magnetic element with respect to the second transceiver-magnetic element and the second holding-magnetic element enables the transceiver assembly to connect the transceiver device to the holding device via an overall magnetic force that allows the transceiver assembly, in particular the transceiver device, to exert a pressure to the body tissue being consistent within a narrow range for both bald and densely haired regions, ensuring continuous patient comfort.

This is achieved by combining the first transceiver-magnetic element and the first holding-magnetic element that preferably exert an essentially exponentially decaying, perpendicular force to the body tissue, with the second transceiver-magnetic element and the second holding-magnetic element that provide an additional force in a perpendicular direction depending on the position of the transceiver device with respect to the holding device and with respect to the connection direction. In fact, and as will be explained in greater detail below, these first and second transceiver-magnetic elements and holding-magnetic elements are preferably arranged such as to overcome the limitations of magnets following an inverse-square attraction pattern, meaning that they only have a small working range suitable for measurements on biological tissue as the exerted force is typically either too low or too high. Through the combination of first-magnetic elements and second-magnetic elements, the transceiver assembly can be applied for different users with different hair characteristics without the necessity to replace any part of the transceiver assembly, or it allows to better adapt to individual head/body shapes.

For example, the transceiver assembly should be usable for both subjects with dense/thick hair and subjects without hair. In cases where there is hair present, the inter-magnetic distance of the first transceiver-magnetic element and the first holding-magnetic element should be small to apply a high overall force, while in cases without hair, the same distance should be higher to apply a lower force. However, due to the inverse-square behavior of the first magnetic elements, it is not possible to address both requirements satisfyingly with only the first-magnetic elements. The presence of the second transceiver-magnetic element and the second holding-magnetic element address this challenge by fulfilling twofold function: first, they can in a sense pull the transceiver device down- or upwards depending on the position of the transceiver device in the holding device, changing the overall magnetic force, and secondly, they introduce a lateral force perpendicular to the connection direction, in a sense aligning the transceiver device and holding device. In particular, when there is no hair present, the presence of the first transceiver-magnetic element and the first holding-magnetic element only could exert a too weak overall magnetic force. In such instances, the presence of the second transceiver-magnetic element and the second holding-magnetic element can in a sense pull the transceiver device downward, i.e. towards the body tissue, and increase the overall magnetic force. When there is hair present, the presence of the first transceiver-magnetic element and the first holding-magnetic element only could exert a too strong overall magnetic force. In such instances, the presence of the second transceiver-magnetic element and the second holding-magnetic element can in a sense pull the transceiver device upward, i.e. away from the body tissue, and decrease the overall magnetic force.

The holding device and the transceiver device are preferably separate components which can be connected to each other, preferably detachably connected to each other.

The transceiver device preferably remains connected to the holding device by the overall magnetic force exerted by first and second transceiver-magnetic elements and by the first and second holding-magnetic elements unless said overall magnetic force is overcome by a person operating the transceiver assembly, e.g., medical staff.

It is further preferred that the holding device and the transceiver device can be manufactured and/or stored independently of each other.

The first transceiver-magnetic element and the first holding-magnetic element are preferably configured to exert an attractive magnetic force to one another. Additionally, or alternatively, the second transceiver-magnetic element and the second holding-magnetic element are preferably configured to exert an attractive and/or repulsive magnetic force to one another.

The transceiver assembly is preferably configured to provide a variable pressure to the body tissue that varies with respect to a position of the transceiver device in relation to the holding device and with respect to the connection direction.

That is, the transceiver assembly, in particular the transceiver device, is preferably configured to exert a variable pressure to the body tissue. Said variable pressure preferably changes depending on the position of the transceiver device with respect to the holding device and with respect to the connection direction. Said variable pressure is preferably a consequence of the overall magnetic force exerted by the first and second transceiver-magnetic elements and by the first and second holding-magnetic elements, wherein said overall magnetic force preferably varies with respect to a position of the transceiver device in relation to the holding device and with respect to the connection direction. Hence, the first and second transceiver-magnetic elements and the first and second holding-magnetic elements are preferably arranged such, that a variable overall magnetic force is generated that varies with respect to a position of the transceiver device in relation to the holding device and with respect to the connection direction. In other words, the combined magnetic forces exerted by the first and second transceiver-magnetic elements and the first and second holding-magnetic elements are preferably used to generate a variable pressure on the body tissue.

Another advantage of said varying overall magnetic field and the variable pressure, respectively, is a reduction or even elimination of motion artefacts to prevent loss of contact and maintain optimal pressure during movement or movement-induced accelerations of a person wearing the transceiver assembly.

The transceiver device is preferably movably connectable to the holding device with respect to the connection direction via the overall magnetic force, and wherein the transceiver device is preferably movable with respect to the connection direction. As such, the transceiver device is configured to adapt different positions with respect to the holding device and with respect to the connection direction.

The first and second transceiver-magnetic elements and the first and second holding-magnetic elements are preferably arranged such, that their overall magnetic force is a linear combination of i) the magnetic force exerted between the first transceiver-magnetic element and the first holding-magnetic element and ii) the magnetic force exerted between the second transceiver-magnetic element and the second holding-magnetic element.

In other words, the first and second transceiver-magnetic elements and the first and second holding-magnetic elements are preferably arranged such that their overall magnetic force varies linearly with respect to a distance between the first transceiver-magnetic element and the first holding-magnetic element with respect to the connection direction.

The first transceiver-magnetic element and the first holding-magnetic element are preferably arranged such that their exerted magnetic force is i) attracting the transceiver device towards the holding device along the connection direction and/or ii) increasing with decreasing distance between the first transceiver-magnetic element and the first holding-magnetic element and with respect to the connection direction in an essentially exponential manner.

It should be noted that the essentially exponential manner or behavior mentioned herein, i.e. an increasingly stronger force with decreasing distance, is understood as following mathematical functions that have essentially exponential behavior in parts of their domain, such as inverse square, hyperbolic or squared-hyperbolic behavior. This reflects also that an exponential or reciprocal exponential function may be approximated by a series of polynomials to a certain extent.

The second transceiver-magnetic element and the second holding-magnetic element are preferably arranged such that, depending on a position of the transceiver device with respect to the holding device and with respect to the connection direction, their magnetic force is attracting the transceiver device towards the holding device along the connection direction or repelling the transceiver device away from the holding device along a disconnection direction extending opposite to the connection direction.

That is to say, the first and second transceiver-magnetic elements and the holding-magnetic elements are preferably arranged such, that their attractive forces provide a magnetic force profile with a highest magnetic force acting on the body tissue occurring at small distances between the holding device and the transceiver device with respect to the connection direction.

The first transceiver-magnetic element and the first holding-magnetic element are preferably arranged at least partially and possibly entirely overlapping with one another and with respect to the connection direction. Additionally or alternatively, the second transceiver-magnetic element and the second holding-magnetic element are preferably arranged at least partially and possibly entirely overlapping with one another and with respect to the transverse direction.

Furthermore, it is preferred that the at least partially overlapping first transceiver-magnetic element and the first holding-magnetic element do not touch each other, in particular not even in a connected state of the transceiver device and the holding device. In other words, the at least partial overlap preferably only exists with regard to a spatial arrangement and/or surface extension of these elements with respect to one another.

To this end it is conceivable that the second magnetic elements of the transceiver device and of the holding device are arranged closer towards an outside of the transceiver assembly than the first magnetic elements of the transceiver device and of the holding device. In this case it is preferred that a size of the holding device is at least in the regions of the magnetic elements larger than a size of the transceiver device. However, the opposite is likewise conceivable, i.e. that the first magnetic elements of the transceiver device and of the holding device are arranged closer toward an outside of the transceiver assembly than the second magnetic elements of the transceiver device and of the holding device. In this case it is preferred that a size of the transceiver device is at least in the regions of the magnetic elements larger than a size of the transceiver device. That is, depending on a size of the holding device and the transceiver device at least in the regions of the magnetic elements an arrangement of the magnetic elements could be interchanged.

The first transceiver-magnetic element preferably extends within a first transceiver plane and the second transceiver-magnetic element extends within a second transceiver plane. The first transceiver plane and the second transceiver plane preferably run at an angle and particularly preferably perpendicularly to one another.

The first holding-magnetic element preferably extends within a first holding plane and the second holding-magnetic element extends within a second holding plane. The first holding plane and the second holding plane preferably run at an angle and particularly preferably perpendicularly to one another.

The first transceiver plane and the first holding plane preferably run parallel to one another and/or the second transceiver plane and the second holding plane preferably run parallel to one another.

A shape of the first and second transceiver-magnetic elements can be the same or different from one another. Likewise, a shape of the first and second holding-magnetic elements can be the same or different from one another. In addition, the shape of the first and/or second transceiver-magnetic elements can be the same or different from the shape of the first and/or second holding-magnetic elements.

Various shapes are conceivable. For example, the first and/or second transceiver-magnetic elements and/or the first and/or second holding-magnetic elements can have the shape of a disc, bar, rectangle, block, horseshoe, round, rings, donuts, segment, or any other desired shape.

The first and/or second transceiver-magnetic elements and/or the first and/or second holding-magnetic elements are preferably permanent magnets. The first and/or second transceiver-magnetic elements and/or the first and/or second holding-magnetic elements preferably comprise or consist of at least one ferromagnetic material such as ferrite or alnico. However, other types of magnetic elements are likewise conceivable and well-known in the art. For example, the first and/or second transceiver-magnetic elements and/or the first and/or second holding-magnetic elements could be electromagnets or switchable magnets or any other magnetic element that can generate a magnetic force in the connection or the transverse direction.

The first and/or second transceiver-magnetic elements and/or the first and/or second holding-magnetic elements are preferably commercially available. For example, these magnetic elements could be Neodymium N52 magnets or Neodymium N42 magnets. In this respect it is noted that N52 magnets have a 20% higher attraction than N42 magnets, which can affect a dimension of the transceiver assembly. In general, the higher the magnetic strength of the first and second transceiver-magnetic elements and the holding-magnetic elements, the smaller a dimension of the transceiver assembly can become.

The first transceiver-magnetic element and/or the second transceiver-magnetic element and/or the first holding magnetic element and/or the second holding magnetic element preferably are dipoles with two magnetic poles that are commonly referred to as a "south pole" and a "north pole".

The first transceiver-magnetic element and the first holding-magnetic element are preferably arranged such that the poles of opposite polarity are opposite to each other with respect to the connection direction. For example, the first transceiver-magnetic element can be arranged in the transceiver device in such a way that its south pole is opposite the north pole of the first holding-magnetic element or vice versa with respect to the connection direction.

The second transceiver-magnetic element and the second holding-magnetic element are preferably arranged such that the poles of opposite polarity are opposite to each other with respect to the transverse direction. For example, the second transceiver-magnetic element can be arranged in the transceiver device in such a way that its south pole is opposite the north pole of the first holding-magnetic element or vice versa with respect to the transverse direction.

The first and second transceiver plane as well as the first and second holding plane preferably run parallel to a plane that separates the north pole and the south pole in the respective first and second transceiver-magnetic elements and the holding-magnetic elements, respectively. For instance, the first transceiver plane associated with the first transceiver-magnetic element preferably runs parallel to a plane separating the north pole and the south pole of said first transceiver-magnetic element.

The transceiver device preferably comprises two or more first transceiver-magnetic elements and/or two or more second transceiver-magnetic elements. The holding device preferably comprises two or more first holding-magnetic elements and/or the two or more second holding-magnetic elements.

Two or more of the first transceiver-magnetic elements are preferably arranged within a common plane, preferably within the first transceiver plane mentioned above, and/or at a distance from one another and/or are arranged opposite one another with respect to a plane running perpendicularly to the transceiver device and parallel to the connection direction.

Two or more of the second transceiver-magnetic elements are preferably arranged within a common plane and/or at a distance from one another and/or are arranged opposite one another with respect to a plane running perpendicularly to the transceiver device and parallel to the connection direction.

Two or more of the first holding-magnetic elements are preferably arranged within a common plane, preferably the first holding plane mentioned above, and/or at a distance from one another and/or are arranged opposite one another with respect to a plane running perpendicularly to the holding device and parallel to the connection direction.

Two or more of the second holding-magnetic elements are preferably arranged within a common plane and/or at a distance from one another and/or are arranged opposite one another with respect to a plane running perpendicularly to the holding device and parallel to the connection direction.

Moreover, one or more of the first and/or second transceiver-magnetic element(s) and/or one or more of the first and/or second holding-magnetic elements can be formed from two or more sub-magnetic elements. Said two or more sub-magnetic elements can be arranged immediately adjacent to one another or at a distance from one another. Said two or more sub-magnetic elements can have a same design such as shape or size or a different design. For example, a second holding-magnetic element could be formed from two second sub-holding-magnetic elements, wherein said second sub-holding-magnetic elements are arranged immediately adjacent to one another and in particular in surface contact with one another when seen along the transverse direction. The provision of such sub-magnetic elements allows a further optimization of the overall magnetic force by locally increasing or decreasing the force along the connection direction depending on the position. That is, the second magnet elements can be seen as helping to pull more strongly at long transceiver-holder distances and to push more strongly against the inverse-square behaviour of the magnetic elements along the connection direction for short distances, and in-between, it can be seen as helping to linearize the overall magnetic force.

A number and/or size of the first transceiver-magnetic element(s) can be the same or different from a number and/or size of the first holding-magnetic element(s). A number and/or size of the second transceiver-magnetic element(s) can be the same or different from a number and/or size of the second holding-magnetic element(s). A number and/or size of the first transceiver-magnetic element(s) can be the same or different from a number and/or size of the second transceiver-magnetic element(s). A number and/or size of the first holding-magnetic element(s) can be the same or different from a number and/or size of the second holding-magnetic element(s). The size is understood as a thickness (e.g. along a z-spatial direction) and a surface area (e.g. along x, y-spatial directions) of the first and second transceiver-magnetic element(s) and the holding-magnetic element(s). Explanations regarding one first and second transceiver-magnetic element and holding-magnetic element preferably likewise apply to two or more first and second transceiver-magnetic elements and holding-magnetic elements and vice versa.

The holding device preferably comprises an insertion opening. The transceiver device is preferably at least partially receivable in said insertion opening. Additionally or alternatively, the transceiver device is preferably movably held within the insertion opening via the overall magnetic force and preferably movable with respect to the connection direction.

However, it is likewise conceivable that the transceiver device comprises an insertion opening. The holding device is at least partially receivable in said insertion opening. Additionally or alternatively, the transceiver device is movably held with respect to the holding device being at least partially received within its insertion opening via the overall magnetic force and preferably movable with respect to the connection direction. Whether the holding device or the transceiver device comprises the insertion opening is preferably determined by their size. Reference is also made to the previous explanations regarding the arrangement of the first and second magnetic elements with respect to an outside of the transceiver assembly.

In the following, explanations are made with respect to the former design, i.e. the holding device comprising the insertion opening. It should be noted that these explanations apply to the case where the transceiver device comprises the insertion opening in an analogous manner. Hence, the transceiver device is preferably connectable to the holding device by inserting the transceiver device into the insertion opening of the holding device. The holding device preferably comprises a holding-housing that comprises said insertion opening. The transceiver device preferably comprises a transceiver-housing that is at least partially received in the holding-housing upon insertion of the transceiver device into the insertion opening of the holding device. Upon insertion of the transceiver device into the insertion opening of the holding device, the transceiver device is preferably connected to the holding device by the overall magnetic force exerted by the first and second transceiver-magnetic elements and by the first and second holding-magnetic elements.

As mentioned initially, the transceiver device is preferably removably connectable to the holding device along the connection direction. To this end it is preferred that the transceiver device is configured to perform a translatory movement in the connection direction and in the disconnection direction, i.e., towards and away from the holding device.

The transceiver device and the holding device preferably comprise in each case at least one fastening element for removably fastening the transceiver device to the holding device. Said fastening can be provided in addition to the magnetic connection. The fastening preferably is a mechanical fastening and/or the fastening elements are preferably configured to enter a positive-locking fit and/or a friction fit. For instance, the fastening elements can be configured to enter a snap-in connection.

The fastening elements are preferably provided on and/or in the holding-housing and the transceiver-housing, for instance in the insertion opening. However, other arrangements of the fastening elements are likewise conceivable. In fact, the fastening elements could be provided anywhere on the transceiver device and the holding device as long as they allow the transceiver device and the holding device being removably fastened to one another.

The transceiver device is preferably rotatably connectable to the holding device and is preferably rotatable about a rotation axis running parallel to the connection direction.

That is, during insertion and/or after insertion of the transceiver device into the insertion opening of the holding device, the transceiver device is preferably rotatable with respect to the holding device.

The transceiver device is preferably rotatable about the rotation axis by a rotation angle of ± 5° or more, such as ± 10° or more, for instance of ± 30° or more.

To this end it is preferred that the holding device and the transceiver device, in particular the holding-housing and the transceiver-housing, in each case comprise at least one rotational stop element such as corresponding protrusions that are configured to abut against one another and delimit a rotation of the transceiver device with respect to the holding device. However, it is likewise conceivable that no rotational stop elements or the like are present, and that the transceiver device can perform a full rotation about the rotation axis.

The transceiver assembly preferably further comprises at least one contacting element being configured to contact the body tissue. The transceiver assembly is preferably configured to press the contacting element against the body tissue via the overall magnetic force. The contacting element is preferably provided on the transceiver device and is preferably protruding from the transceiver device along the connection direction. The holding device preferably comprises at least one aperture through which the contacting element can at least partially protrude when contacting the body tissue.

The contacting element preferably has the shape of a protruding tip or the like that protrudes from a bottom side of the transceiver device. When the transceiver device is connected to the holding device, in particular when the transceiver device is at least partially inserted into the insertion opening of the holding device, the contacting element preferably protrudes at least partially from the aperture of the holding device so as to contact the body tissue.

The transceiver device preferably comprises at least one transmitter device being configured to transmit the signal to the body tissue and/or at least one receiver device being configured to receive a signal from the body tissue.

The signal being transmitted to the body tissue and/or being received from the body tissue preferably is an optical signal and/or an electrical signal and/or an electromagnetic signal and/or an acoustic signal.

The transceiver assembly is particularly preferably configured for receiving signals of a body tissue and/or transmitting signals to a body tissue in a non-invasive manner.

The transmitter device preferably is at least one of an electrode or a transducer such as an optical transducer, an acoustic transducer, an ultrasonic transducer, or an electromagnetic transducer.

The signal being transmitted from the transmitter device to the body tissue preferably is at least one of an electrical signal, an optical signal, an acoustic signal, an ultrasonic signal, or an electromagnetic signal.

The signal being received from the body tissue preferably is at least one of an electrical signal, an optical signal, an acoustic signal, an ultrasonic signal, or an electromagnetic signal.

The signal being received from the body tissue preferably is based on the signal being transmitted from the transmitter device to the body tissue. For instance, the transmitter device can transmit a signal to the body tissue that is reflected from the body tissue and thereafter received by the receiver device.

The receiver device preferably is configured to receive at least one of an electrical signal, an optical signal, an acoustic signal, an ultrasonic signal or an electromagnetic signal and is, for instance a light detector such as a Silicon Photomultiplier (SiPM), a photodiode (PD), an avalanche photodiode (APD) or a photomultiplier tube (PMT).

The transceiver assembly can comprise two or more transceiver devices, wherein these two or more transceiver devices are arranged in an array. Likewise, the transceiver assembly can comprise two or more receiver devices that are arranged in an array.

To this end it is particularly preferred that the transceiver assembly is configured to perform at least one of electroencephalography (EEG) measurements, electromyography (EMG) measurements, photoplethysmography (PPG) measurements, optical coherence tomography (OCT), ultrasound imaging (USI) or Near-infrared spectroscopy (NIRS) measurement.

As such, it is preferred that the transmitter device is at least one of an electrode for electroencephalography (EEG) and electromyography (EMG), an optical transducer for photoplethysmography (PPG) or for Near-infrared spectroscopy (NIRS) and optical coherence tomography (OCT), or an acoustic/ultrasonic transducer for ultrasound imaging (USI).

The transceiver assembly, in particular the transceiver device, preferably further comprises and/or is in connection with at least one analyzing device for analysing the signal of the body tissue being received by the transceiver assembly.

However, it should be noted that the transceiver assembly can likewise be configured and/or be used for transmitting signals to a body tissue only. For example, the transceiver assembly can be used or can be configured to transmit signals to a body tissue, for instance into a body tissue, such as in non-invasive red-light therapy or in electrical/electromagnetic stimulations, for instance in transcranial-direct-current stimulations.

Furthermore, the transceiver assembly might not only be configured for or used in the medical field but it can find application in other fields, for instance in virtual reality (VR). For example, the transceiver assembly could comprise or be provided in VR goggles to allow for a stable and comfortable placement of a subpart of the VR device, such as the screen or an eye-tracking camera. Other examples involve its use for headphones to enable comfortable placement, or for smartwatches or medical oximeters to enable good optical/PPG measurements, or for the wireless charging of an implanted device, such as an implanted EEG array or a neurostimulator.

The transmitter device and/or the receiver device are preferably at least partially arranged in the contacting element.

It is conceivable that both, the at least one transmitter device and the at least one receiver device, are at least partially arranged in a common contacting element. However, it is likewise conceivable that the transceiver assembly, in particular the transceiver device, comprises at least two contacting elements, wherein one of the contacting elements comprises the at least one transmitter device and the other of the contacting elements comprises the at least one receiver device.

For example, one of the contacting elements can comprise a transmitter device in the form of a light source and the other contacting element can comprise one or more receiver devices in the form of a light detector or a light detector array. For example, said other contacting element could comprise a light detector array comprising a Silicon Photomultiplier (SiPM) and/or a photodiode (PD). The SiPM is preferably used for small light intensity measurements in biological tissue, for example with lateral distances between the contacting device comprising the transmitter device and the contacting element comprising the receiver device being around 30 millimeters, and the PD is preferably used for the short-distance measurements with high light intensities, for example with lateral distances between the transmitter device and the receiver device being around 8 millimeters. Said distances between the transmitter device and the receiver device are commonly referred to as source-detector separations.

As will be explained in greater detail below, the transceiver assembly preferably comprises a plurality of holding devices and transceiver devices, which consequently preferably also comprise a plurality of transmitter devices and receiver devices. A distance between two or more of these transmitter devices and receiver devices can be the same or different from one another. In other words, a source-detector separation can be the same or different. Again in other words, the transmitter devices and receiver devices are preferably arranged and/or configured for short-distance and/or long-distance measurements. In fact, the transceiver assembly is preferably configured to perform multi-distance measurements, i.e. receive signals of a body tissue and/or transmit signals to a body tissue over multi-distances. As such, it is preferred that the transmitter devices and the receiver devices are arranged and/or configured for both, short-distance and long-distance measurements. It is furthermore preferred that the transceiver assembly is configured to perform such multi-distance measurements concomitantly, i.e. at the same time.

The transceiver assembly preferably further comprises at least one attaching device for attaching the holding device to a body part of a human being or an animal. The attaching device preferably is a headwear such as a cap, a headband, or a headgear such as a virtual-reality headgear, or a bodywear being attachable to a trunk or a limb.

The holding device is preferably attached to the attaching device and via the attaching device attachable to a body part of a human being or an animal whose body tissue is receiving the signals being transmitted by the transceiver assembly and/or whose signals are received by the transceiver assembly.

There are many ways the attaching device and the holding device are connected to one another. For instance, the attaching device can comprise a recess or the like into which the holding device is received. The holding device can be glued or snapped into the attaching device, for instance into a recess of the attaching device. However, it is likewise conceivable that the attaching device and the holding device are manufacture in one piece.

As mentioned earlier, it is particularly preferred that the transceiver assembly comprises a plurality of holding devices and a plurality of transceiver devices. Statements made with respect to one holding device preferably likewise apply to two or more holding devices and vice versa. Statements made with respect to one transceiver device preferably likewise apply to two or more transceiver devices and vice versa.

In another aspect, a method of manufacturing a transceiver assembly for receiving signals of a body tissue and/or transmitting signals to a body tissue, preferably a transceiver assembly as described above, is provided. The method comprises the steps of i) providing at least one holding device, and ii) providing at least one transceiver device. The transceiver device is configured to receive a signal from a body tissue and/or to transmit a signal to a body tissue. The transceiver device is connectable to the holding device along a connection direction. The transceiver device comprises at least one first transceiver-magnetic element and the holding device comprises at least one first holding-magnetic element. The first transceiver-magnetic element and the first holding-magnetic element are arranged after one another with respect to the connection direction and being configured to exert a magnetic force to one another. The transceiver device comprises at least one second transceiver-magnetic element and the holding device comprises at least one second holding-magnetic element. The second transceiver-magnetic element and the second holding-magnetic element are arranged after one another with respect to a transverse direction running perpendicularly to the connection direction and are configured to exert a magnetic force to one another. In that context, two magnetic elements being arranged after one another indicates that the magnetic polarities of the two magnetic elements have an interaction among each other which exerts a force behavior as described in the previous passages, describing an essentially exponential behavior in one direction and a position-dependent behavior in another direction. Advanced manufacturing processes could allow the production of magnetic elements compromising the first-magnetic and second-magnetic elements in one element, exerting magnetic forces in both directions of along and perpendicular to the connection direction. Moreover, the first-magnetic elements among themselves and/or the second-magnetic elements among themselves must not be exactly parallel, but it is conceivable that an angle between them is possible, as long as the previously described effect of the combination of their magnetic forces is achieved.

The transceiver device is connectable to the holding device via an overall magnetic force exerted by the first and second transceiver-magnetic elements and by the first and second holding-magnetic elements.

Any statements made with respect to the transceiver assembly preferably likewise apply to the method of manufacturing a transceiver assembly and vice versa.

The transceiver assembly, in particular the holding device and/or the transceiver device, can be 3D printed or injection molded, for instance. The first and second transceiver-magnetic elements and/or the first and second holding-magnetic elements are preferably adhesively attached, such as glued to the transceiver device and the holding device, respectively. Other ways such as injection molding the magnetic elements to the transceiver and holding devices are likewise conceivable.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1a: shows a perspective view of a transceiver assembly comprising an attaching device for attachment to a body part of a human being or an animal, a holding device and a transceiver device;
- Fig. 1b: shows a front view of the transceiver assembly according to figure 1a;
- Fig. 2: shows a perspective view of the holding device and the transceiver device of the transceiver assembly according to figure 1a, wherein the transceiver device is partially inserted into the holding device;
- Fig. 3: shows a perspective view of the holding device and the transceiver device of figure 1a in a first position;
- Fig. 4: shows a perspective view of the holding device and the transceiver device of figure 1a in a second position;
- Fig. 5: shows an exploded view of the transceiver device of the transceiver assembly of figure 1a;
- Fig. 6: shows an exploded view of the holding device of the transceiver assembly of figure 1a;
- Fig. 7a: shows a partial sectional view of the holding device and the transceiver device being in the first position according to figure 3;
- Fig. 7b: shows another partial sectional view of the holding device and the transceiver device being in the first position according to figure 3;
- Fig. 7c: shows a sectional view of the holding device and the transceiver device being in the first position according to figure 3;
- Fig. 8a: shows a partial sectional view of the holding device and the transceiver device being in the second position according to figure 4;
- Fig. 8b: shows another partial sectional view of the holding device and the transceiver device being in the second position according to figure 4;
- Fig. 8c: shows a sectional view of the holding device and the transceiver device being in the second position according to figure 4;
- Fig. 9: shows a partial exploded view of the transceiver assembly, wherein the transceiver device comprises a transmitter device, a receiver device and an analysing device;
- Fig. 10: shows a partial sectional view of the transceiver assembly according to figure 1a being attached to a body part, wherein the holding device and the transceiver device are in the first position;
- Fig. 11: shows a partial sectional view of the transceiver assembly according to figure 1a being attached to a body part, wherein the holding device and the transceiver device are in the second position;

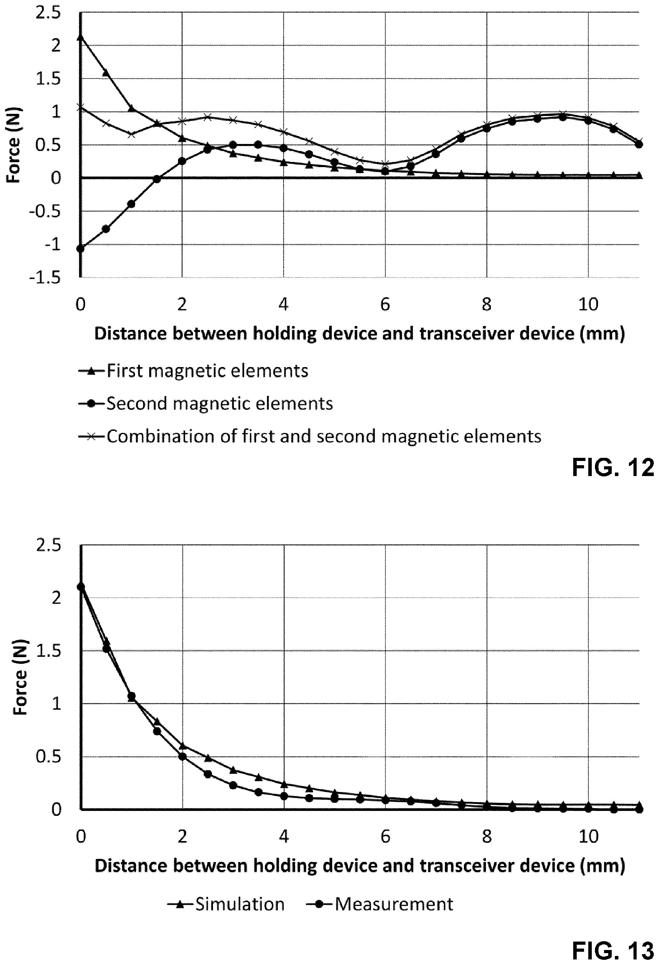

- Fig. 12: shows a graph depicting the magnetic forces of one configuration of the invention, compromising two first and two second holding-magnetic elements and two first and two second transceiver-magnetic elements, whereas each second transceiver-magnetic element compromises one larger magnet and one smaller magnet. The force curves are depicted for the first and second magnetic elements separately as well as their combination. A distance of 0 mm corresponds to a minimal distance between transceiver device and holding device, and increasing distances correspond to an increased distance in disconnection direction;
- Fig. 13: shows a graph depicting a comparison between simulations of the force exerted by the first magnetic elements and a force measurement of a bench setup;
- Fig. 14: shows a graph depicting a comparison between simulations of the force exerted by the second magnetic elements and a force measurement of a bench setup;
- Fig. 15: shows a graph depicting simulations of different second magnetic elements, including second holding-magnetic elements compromised of one small magnet (4mm x 4mm x 1mm), one large magnet (10mm x 4mm x 1mm) and a combination of these two magnets with the larger magnet being positioned towards the transceiver side.

### DESCRIPTION OF PREFERRED EMBODIMENTS

Aspects of the transceiver assembly 1 according to the invention are now discussed with respect to the figures.

In particular, the transceiver assembly 1 according to the invention is configured for receiving signals of a body tissue 2 and/or transmitting signals to a body tissue 2.

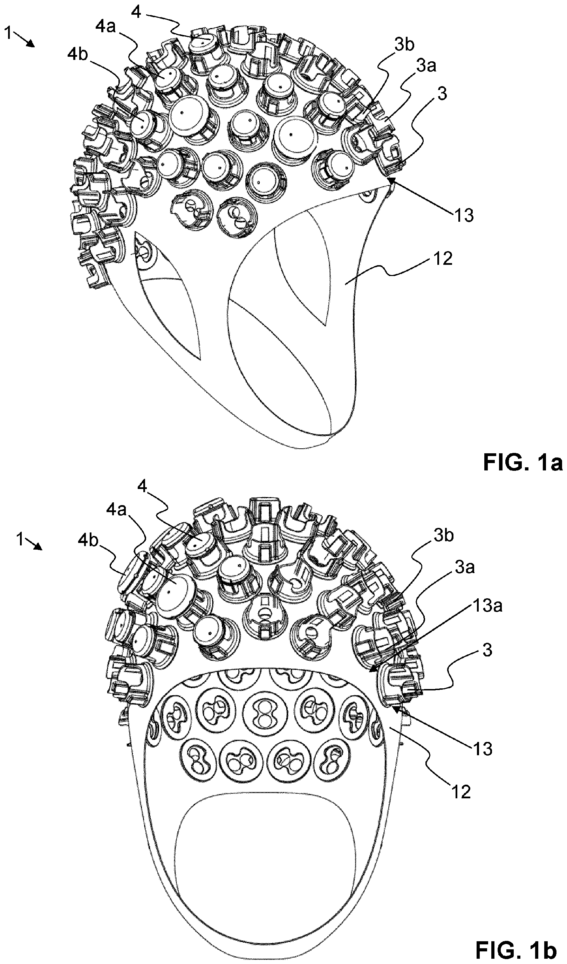

As follows from figures 1a and 1b, the transceiver assembly 1 comprises a plurality of holding devices 3, 3a, ... and a plurality of transceiver devices 4, 4a, ..., wherein the transceiver devices 4, 4a, ... are configured to receive a signal from a body tissue 2 and/or to transmit a signal to a body tissue 2. The transceiver assembly 1 further comprises an attaching device 12 for attaching the holding devices 3, 3a, ... to a body part of a human being or an animal. In the depicted examples, the attaching device 12 is a headwear, in particular a cap that can be worn by a human being whose body tissue 2 is receiving the signals being transmitted by the transceiver assembly 1 and/or whose signals are received by the transceiver assembly 1. The holding devices 3, 3a, ... are fixedly attached to the attaching device 12, namely here snapped into recesses 13, 13a, ... of the attaching device 12.

In the following, explanations are usually made with respect to one holding device 3 and one transceiver device 4 for the sake of simplicity. It should be noted that these explanations likewise apply to the plurality of holding devices 3, 3a, ... and the plurality of transceiver devices 4, 4a, ....

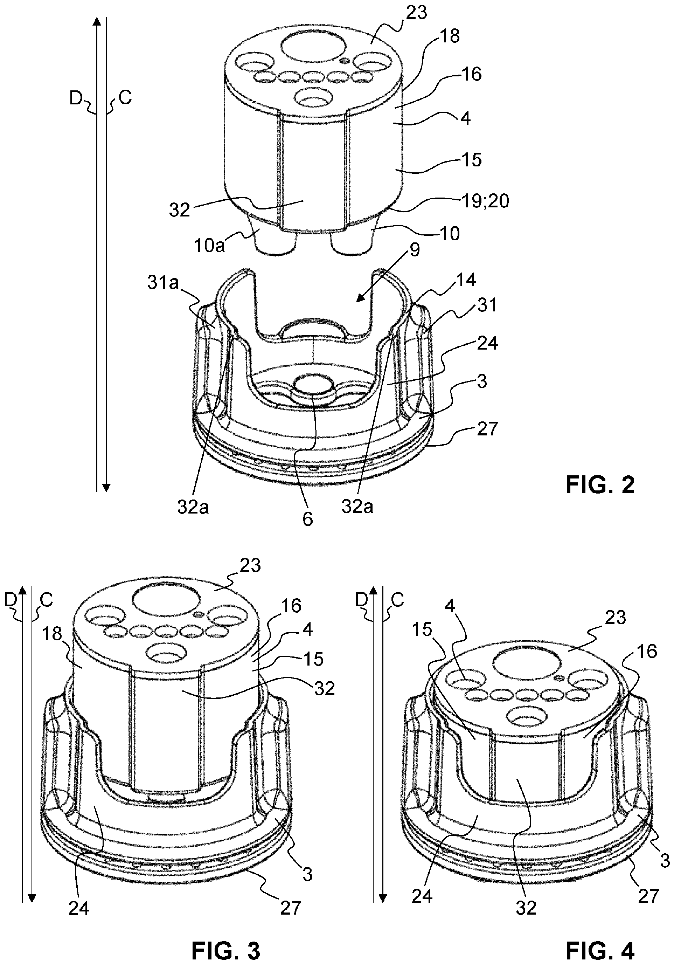

As becomes apparent from figure 2, the holding device 3 and the transceiver device 4 are separate components which can be detachably connected to each other. To this end the transceiver device 4 is connectable to the holding device 3 along a connection direction C, wherein the holding device 3 comprises a holding-housing 14 that defines an insertion opening 9 into which the transceiver device 4 is inserted. During connection or insertion of the transceiver device 4, the transceiver device 4 performs a translatory movement in the connection direction C and towards the holding device 3. If the transceiver device 4 is to be disconnected from the holding device 3, it is moved along an opposite direction, i.e., along a disconnection direction D and away from the holding device 3.

The transceiver device 4 comprises a transceiver-housing 15 that is at least partially received in the holding-housing 14 upon insertion of the transceiver device 4 into the insertion opening 9 of the holding device 3. As will be explained in greater detail further below, upon insertion of the transceiver device 4 into the insertion opening 9 of the holding device 3, the transceiver device 4 is connected to the holding device 3 by an overall magnetic force exerted by first and second transceiver-magnetic elements 5, 7 and by first and second holding-magnetic elements 6, 8. Furthermore, the transceiver device 4 is movably held within the insertion opening 9 via said overall magnetic force and is movable with respect to the connection direction C and the disconnection direction D.

As furthermore follows from these figures, the transceiver-housing 15 comprises a circumferential side wall 16, which is open at the top side 18 and forms an opening 17. On the opposite bottom side 19, the transceiver-housing 15 has a bottom wall 20, which has recesses 21, 21a for receiving the first transceiver-magnetic elements 5, 5a. The bottom wall 20 also has two contacting elements 10, 10a for contacting the body tissue 2, the function of which will be explained in more detail later. The circumferential side wall 16 has recesses 22, 22a in which the second transceiver-magnetic elements 7, 7a are accommodated. The opening 17 at the top side 18 of the transceiver-housing 15 is closable by a lid 23.

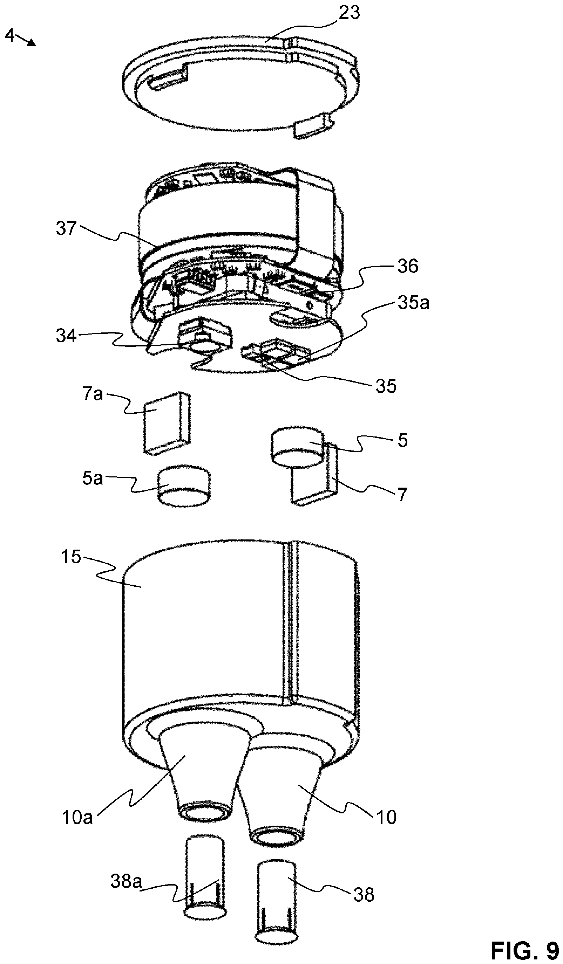

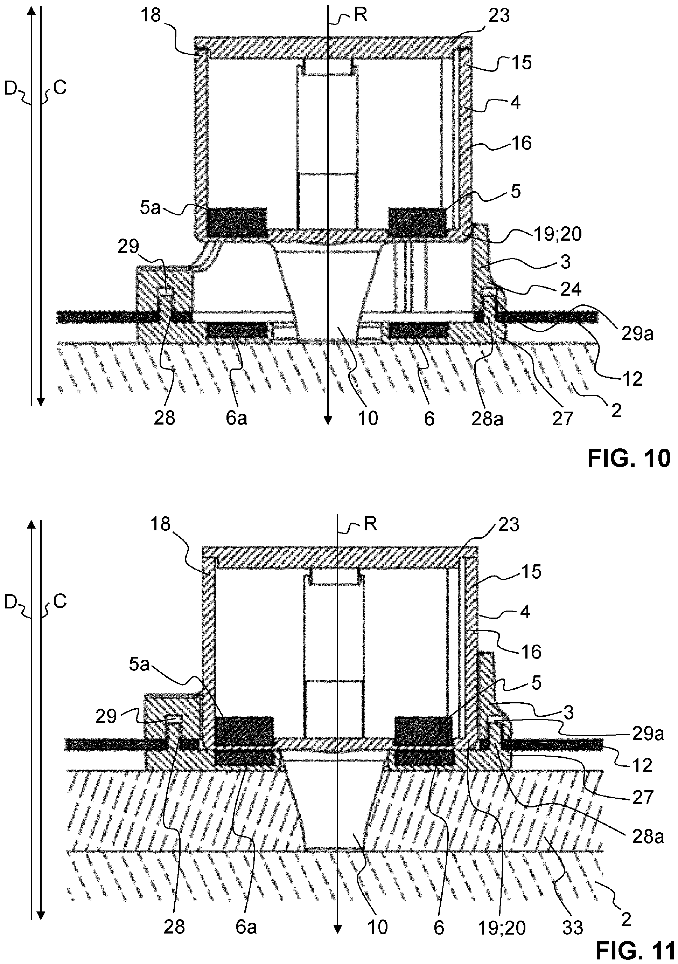

The holding-housing 14 also has a circumferential side wall 24, which in this case is open both at a top side 25 and a bottom side 26. The bottom side 26 of the side wall 24 can be connected to a bottom wall 27, which has a plurality of pins 28, 28a, .... These pins 28, 28a, ... can be accommodated in corresponding apertures 29, 29a, ... in the bottom side 19 of the side wall 24, whereby the bottom wall 27 is connectable to the side wall 24 of the holding-housing 14 in a force-fit and/or form-fit manner, see in particular figures 9 and 10. Furthermore, the bottom wall 27 of the holding-housing 14 has a dumbbell-shaped aperture 11 through which the contacting elements 10, 10a of the transceiver device 4 can at least partially protrude. The bottom wall 27 of the holding-housing 14 also has two recesses 30, 30a in which the first holding-magnetic elements 6, 6a are accommodated. The second holding-magnetic elements 8, 8a are arranged in corresponding recesses 31, 31a of the side wall 24 of the holding-housing 14.

The transceiver-housing 15 is rotatably receivable in the holding-housing 14 and is rotatable about a rotation axis R running parallel to the connection direction C. This rotatability is due, among other things, to the essentially round design of the transceiver-housing 15 and holding-housing 14 when viewed in cross-section. Furthermore, the transceiver-housing 15 and holding-housing 14 in the examples have rotational stop elements that restrict the angle of rotation, see the rotational stop element 32 of the transceiver device in the form of a bulge in figures 2-5 and the rotational stop element 32a of the holding device in figure 2.

In fact, the round design also allows for twisting the transceiver device 4 within the holding device 3. This advantageous feature simplifies donning as it enables easier accommodation of hair 33 on the body tissue 2 to be probed, for instance, see also further below. While special tools are often needed to brush away hairs or rotate the transceiver devices 4 through hair, the transceiver assembly 1 according to the invention eliminates this necessity. In particular, by grasping the transceiver device 4 with two fingers, it can be simply rotated onto the hair, allowing the contacting elements 10, 10a to probe through the hair. This significantly streamlines the attachment process and reduces the required donning time.

As a result, the transceiver assembly 1 according to the invention enables a straightforward attachment of transceiver devices 4, 4a, ...to probing body tissue 2 such as the skin, generating a controlled force that provides an optimal balance between comfort and signal quality while accommodating various hair conditions. This solution greatly enhances the usability of any system, improving signal quality, setup time, and overall comfort.

The transceiver device and the holding device can comprise in each case at least one fastening element for removably fastening the transceiver device to the holding device. Said fastening can be provided in addition to the magnetic connection. The fastening preferably is a mechanical fastening and/or the fastening elements are preferably configured to enter a positive-locking fit and/or a friction fit. For instance, the fastening elements can be configured to enter a snap-in connection.

The fastening elements are preferably provided on and/or in the holding-housing and the transceiver-housing, for instance in the insertion opening. However, other arrangements of the fastening elements are likewise conceivable. In fact, the fastening elements could be provided anywhere on the transceiver device and the holding device as long as they allow the transceiver device and the holding device being fastened to one another.

As mentioned earlier, the transceiver device 4 is connectable to the holding device 3 via an overall magnetic force which enables the transceiver device 4 to adopt different positions with respect to the holding device 3 and with respect to the connection direction C, compare for instance figures 3 and 4. Namely, figure 3 depicts a first position, wherein the transceiver device 4 is located further away from the holding device 3 with respect to the connection direction C as compared to the second position depicted in figure 4.

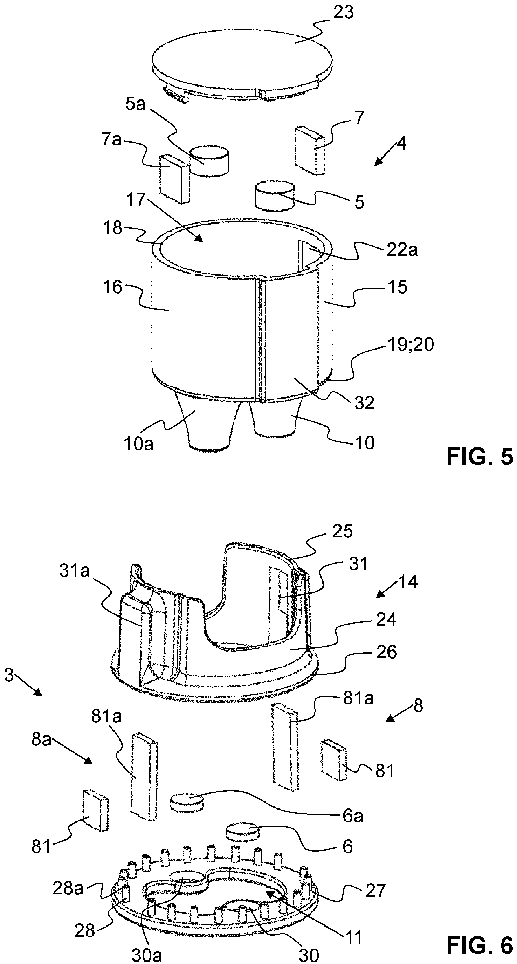

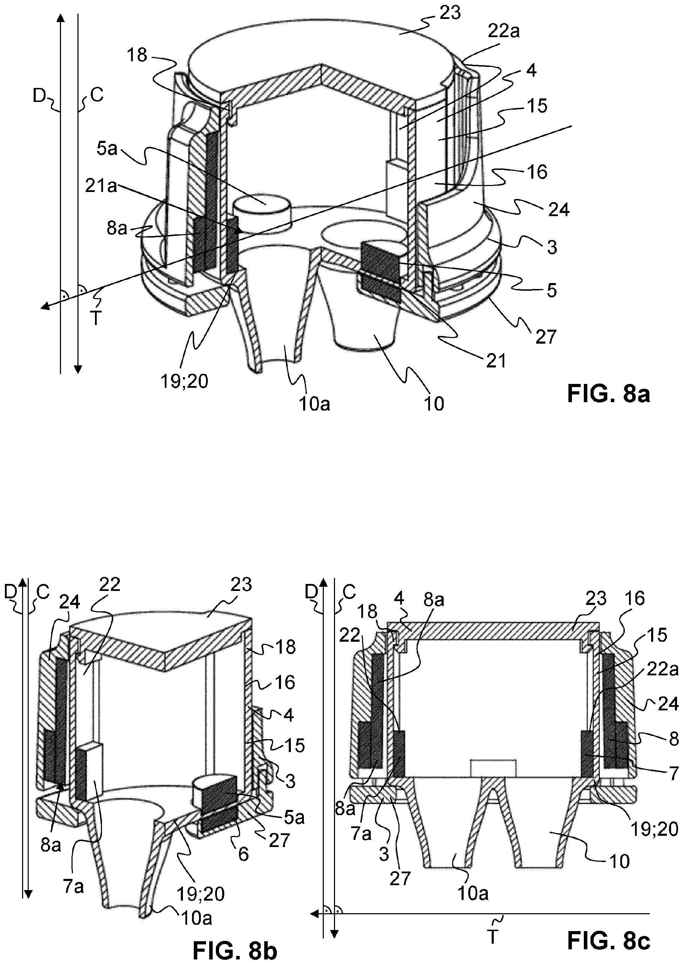

Said overall magnetic force is exerted by the first and second transceiver-magnetic elements 5, 5a; 7, 7a and by the first and second holding-magnetic elements 6, 6a; 8, 8a, see figures 5 to 8c.

That is, the transceiver device 4 comprises here two first transceiver-magnetic elements 5, 5a and the holding device 3 comprises two first holding-magnetic elements 6, 6a. The first transceiver-magnetic elements 5, 5a and the first holding-magnetic elements 6, 6a are arranged after one another with respect to the connection direction C and are configured to exert a magnetic force to one another. The transceiver device 4 comprises here two second transceiver-magnetic elements 7, 7a and the holding device 3 comprises two second holding-magnetic elements 8, 8a. The second transceiver-magnetic elements 7, 7a and the second holding-magnetic elements 8, 8a are arranged after one another with respect to a transverse direction T running perpendicularly to the connection direction C and are configured to exert a magnetic force to one another.

One of the first transceiver-magnetic elements 5, 5a and one of the first holding-magnetic elements 6, 6a are in each case arranged entirely overlapping with one another and with respect to the connection direction C. Similarly, one of the second transceiver-magnetic elements 7, 7a and one of the second holding-magnetic elements 8, 8a are in each case arranged partially overlapping with one another and with respect to the transverse direction T. As furthermore follows from these figures, the overlapping first transceiver-magnetic elements 5, 5a and first holding-magnetic elements 6, 6a do not touch each other but the overlap only exists with regard to the spatial arrangement and/or the surface extension of these magnetic elements 5, 5a; 6, 6a with respect to one another.

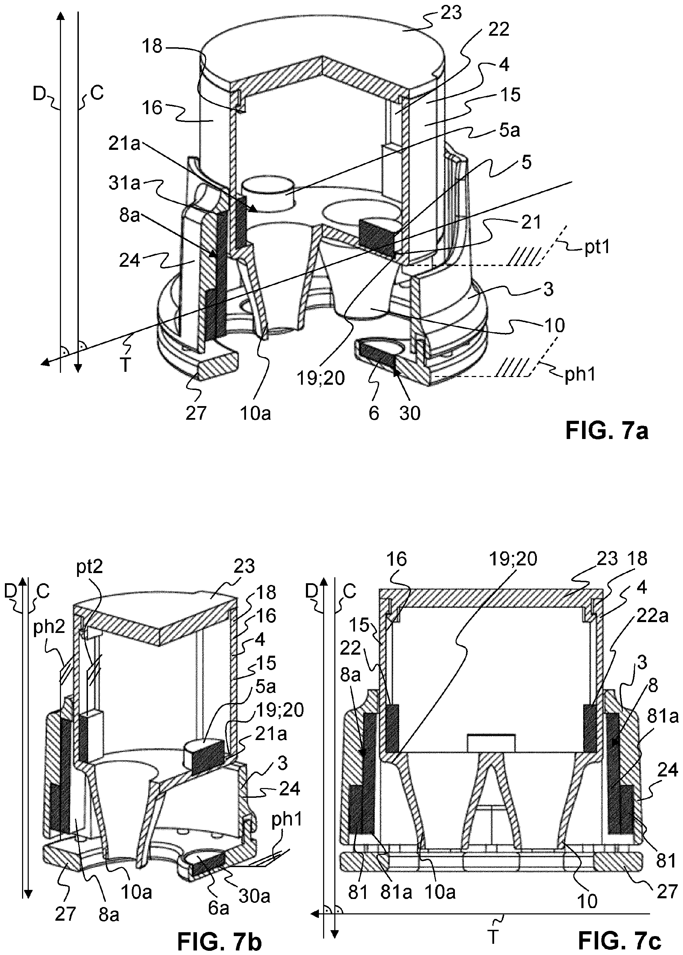

As follows from figures 7a to 8c, the second magnetic elements 7, 7a; 8, 8a of the transceiver device 4 and of the holding device 3 are arranged closer towards an outside of the transceiver assembly 1 than the first magnetic elements 5, 5a; 6, 6a of the transceiver device 4 and of the holding device 3. This is a consequence of the larger size of the holding device 3, which results in the transceiver device 4 being received in the insertion opening 9 of the holding device 3 and not vice versa. However, an opposite design with the transceiver device 4 being larger than the holding device 3 is likewise conceivable although not depicted.

The first transceiver-magnetic elements 5, 5a extend within a first transceiver plane pt1 and the second transceiver-magnetic elements 7, 7a extend within a second transceiver plane pt2, see figures 7a and 7b. Here, said first transceiver plane pt1 and second transceiver plane pt2 run perpendicularly to one another. The first holding-magnetic elements 6, 6a extend within a first holding plane ph1 and the second holding-magnetic elements 8, 8a extend within a second holding plane ph2. Here, said first holding plane ph1 and second holding plane ph2 run perpendicularly to one another. The first transceiver plane pt1 and the first holding plane ph1 run parallel to one another, and the second transceiver plane pt2 and the second holding plane ph2 run parallel to one another in a default state (since they can be rotated). Moreover, it should be noted that the magnetic elements need not have a planar shape but a curve shape instead, for instance. In this case the above explanations with regard to the planes and their arrangement with respect to one another can be understood as applying to a (fictitious) reference plane running through the magnetic elements. Moreover, in the depicted examples a shape of the first and second transceiver-magnetic elements 5, 5a; 7, 7a are different from one another, with the first transceiver-magnetic elements 5, 5a having a disc-like shape and the second transceiver-magnetic elements 7, 7a having a squared shape. The same applies with respect to the first and second holding-magnetic elements 6, 6a; 8, 8a. Furthermore, the first transceiver-magnetic elements 5, 5a and the second transceiver-magnetic elements 7, 7a are diametrically opposite each other with respect to the connection direction C. The same applies with respect to the first and second holding-magnetic elements 6, 6a; 8, 8a.

In the depicted examples, the second holding-magnetic elements 8, 8a are in each case formed from two sub-magnetic elements 81, 81a. That is, each second holding-magnetic element 8, 8a is a two-piece component, with in each case two sub-magnetic elements 81, 81a constituting one of the second holding-magnetic elements 8, 8a. Each of the two sub-magnetic elements 81, 81a constituting one of the second holding-magnetic elements 8, 8a are arranged immediately adjacent to one another with respect to the transverse direction T and are of a different design. In particular, the sub-magnetic element 81 being arranged closer towards the outside of the transceiver assembly 1 is smaller than the sub-magnetic element 81a being arranged towards an inside of the transceiver assembly 1 or towards the insertion opening 9 of the holding device 3, respectively.

A number of the first transceiver-magnetic elements 5, 5a is the same as a number of the first holding-magnetic elements 6, 6a and a number of the second transceiver-magnetic elements 7, 7a is the same as a number of the second holding-magnetic elements 8, 8a, wherein the two sub-magnetic elements 81, 81a constituting a second holding-magnetic element 8, 8a are understood as one second holding-magnetic element 8, 8a. A number of the first transceiver-magnetic elements 5, 5a is the same as a number of the second transceiver-magnetic elements 7, 7a and a number of the first holding-magnetic elements 6, 6a is the same as a number of the second holding-magnetic elements 8, 8a. In particular, the depicted examples comprise two first transceiver-magnetic elements 5, 5a, two second transceiver-magnetic elements 7, 7a, two first holding-magnetic elements 6, 6a and two second holding-magnetic elements 8, 8a.

As mentioned earlier, the transceiver device 4 comprises at least one contacting element 10 being configured to contact the body tissue 2. In the depicted examples, the transceiver device comprises two contacting elements 10, 10a in the form of protruding tips that are formed in the bottom wall 20 of the transceiver-housing 15 and that are arranged next to one another.

In the depicted examples, the first and second transceiver-magnetic elements 5, 5a; 7, 7a and the first and second holding-magnetic elements 6, 6a; 8, 8a are permanent magnets having dipoles with two magnetic poles that are referred to as a "south pole" and a "north pole".

The first transceiver-magnetic elements 5, 5a and the first holding-magnetic elements 6, 6a are arranged such that the poles of opposite polarity are opposite to each other with respect to the connection direction C. Here, the first transceiver-magnetic elements 5, 5a are arranged in the transceiver device 4 in such a way that their south pole is opposite the north pole of the first holding-magnetic elements 6, 6a with respect to the connection direction C. Moreover, the second transceiver-magnetic elements 7, 7a and the second holding-magnetic elements 8, 8a are arranged such that the poles of opposite polarity are opposite to each other with respect to the transverse direction T. Here, the second transceiver-magnetic elements 7, 7a are arranged in the transceiver device 4 in such a way that their south pole is opposite the north pole of the first holding-magnetic elements 6, 6a with respect to the transverse direction T. It is not necessary that the poles of the aforementioned magnets are perfectly aligned.

The first and second transceiver plane pt1, pt2 as well as the first and second holding plane ph1, ph2 run parallel to a plane that separates the north pole and the south pole in the respective first and second transceiver-magnetic elements 5, 5a; 7, 7a and the holding-magnetic elements 6, 6a; 8, 8a, respectively.

Figure 9 depicts the transceiver device 4 as just described, wherein the transceiver device 4 comprises a transmitter device 34 being configured to transmit the signal to the body tissue 2 in the form of a light emitting diode (LED) and two receiver devices 35, 35a being configured to receive a signal from the body tissue in the form of a photodiode (PD) and a Silicon Photomultiplier (SiPM). The transceiver device 4, in particular the receiver devices 35, 35a, are in connection with an analyzing device 36 in the form of a printed circuit board (PCB) for analysing the signal of the body tissue 2 being received by the receiver devices 35, 35a. The transmitter device 34 and the receiver devices 35, 35a are at least partially arranged in the contacting elements 10, 10a. In fact, one of the contacting elements 10 comprises the transmitter device 34 and the other contacting element 10a comprises both receiver devices 35, 35a. The transmitter device 34 further compromises two light transmitting elements 38, 38a to optimally guide the light from the transmitter device 34 to the tissue 2 and, vice versa, from the tissue 2 to the receiver devices 35, 251.The transmitter device 34 and the receiver devices 35, 35a as well as the analysing device 26 are each in connection with a power source 37 in the form of a battery that powers these components.

As illustrated in figures 10 and 11, the transceiver assembly 1 is configured to provide a variable pressure to the body tissue 2 that varies with respect to a position of the transceiver device 4 in relation to the holding device 3 and with respect to the connection direction C. In particular, their magnetic elements 5, 5a; 6, 6a; 7, 7a; 8, 8a are arranged and configured such that their overall magnetic force allows the transceiver device 4 to exert a pressure to the body tissue 2 being consistent within a narrow range for both bald haired regions (see figure 10) and densely haired regions (see figure 11).

In fact, the presence of solely the first transceiver-magnetic elements 5, 5a and the first holding-magnetic elements 6, 6a could apply optimal pressure only for a limited operation range, exerting excessively high forces for short inter-magnetic distances such as in subjects with dense hair as indicated in figure 11 or insufficient forces for large inter-magnetic distances for bald subjects as indicated in figure 10. In instances with excessive force, the presence of the second transceiver-magnetic elements 7, 7a and the second holding-magnetic elements 8, 8a pull the transceiver device 4 upwards, i.e. away from the body tissue 2, reducing the overall magnetic force. In instances with insufficient force, the presence of the second transceiver-magnetic elements 7, 7a and the second holding-magnetic elements 8, 8a pull the transceiver device 4 downward, i.e. towards the body tissue 2.

Hence, to ensure a robust and comfortable application of the transceiver assembly 1, it is crucial to have a connection mechanism provided by the transceiver device 4 and the holding device 3 and their overall magnetic force that can accommodate the interpersonal differences in hair and anatomy among users, as well as any movements that may occur during the application. To address these sometimes conflicting requirements, a connection mechanism capable of applying well-defined and modular/variable force to the probing body tissue 2 is needed, tailored to each patient's needs.

On one hand, the connection mechanism must possess sufficient flexibility to prevent the occurrence of strong artifacts or significant changes in forces (as high forces can cause discomfort, while low forces can lead to signal loss) caused by movements. On the other hand, the connection mechanism must be capable of exerting the before-mentioned consistent force within a narrow range for both bald and densely haired regions, ensuring continuous patient comfort.

In the present invention, this is achieved by combining the first transceiver-magnetic elements 5, 5a and first holding-magnetic elements 6, 6a that exert an inverse-squarely decaying, magnetic force along the connection direction C with second transceiver-magnetic elements 7, 7a and second holding-magnetic elements 8, 8a that provide an additional force along the connection direction depending on a position of the transceiver device 4 with respect to the holding device 3. The linear combination of these downward and upward forces allows to the transceiver assembly 1 according to the invention to overcome the limitations of magnetic elements following an inverse-square attraction pattern.

Thus, the transceiver assembly 1 according to the invention implements attractive magnetic forces of the magnetic elements 5, 5a; 6, 6a; 7, 7a; 8, 8a to generate a modular/variable pressure on the probing body tissue 2, wherein the first transceiver-magnetic elements 5, 5a and first holding-magnetic elements 6, 6a, which are in a sense horizontally arranged in the transceiver assembly 1 and can thus be seen as horizontal magnetic elements, are combined with the second transceiver-magnetic elements 7, 7a and second holding-magnetic elements 8, 8a, which are in a sense laterally arranged from the first transceiver-magnetic elements 5, 5a and first holding-magnetic elements 6, 6a and can thus be seen as lateral magnetic elements, to overcome the essentially exponential nature of magnetic attraction.

In figure 12, an increase in the overall force along the connection direction C is shown for distances above 6 mm between the transceiver element 4 and the holding element 3This intentional behavior introduces a magnetic edge which needs to be overcome to disconnect the transceiver device 4 from the holding device 3. This facilitates the insertion of the transceiver device 4 into the holding device thanks to a larger attraction force and reduces the risk of a transceiver device 4 to unintentionally being disconnected from the holding device 3 due to movements or a mechanical contact.

As follows from the graph depicted in figure 13, the first transceiver-magnetic elements 5, 5a and first holding-magnetic elements 6, 6a, or horizontal magnetic elements, exert a continuous force along the connection direction C towards the holding device 3 following an exponential manner. This force profile was validated with simulations (triangular marks) and bench measurements (round marks), which depict a good overlap of the force profile and magnitudes.

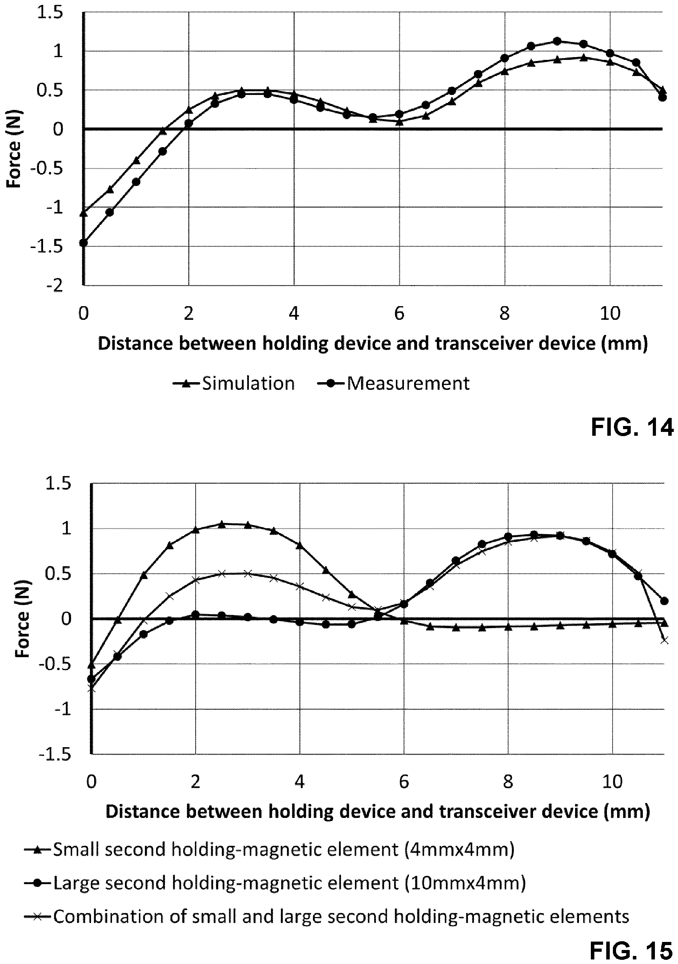

As follows from the graph depicted in figure 14, the second transceiver-magnetic elements 7, 7a and second holding-magnetic elements 8, 8a, or lateral magnetic elements, exert variable forces in connection direction C depending on the current position of the transceiver device 4 within the holding device 3. This force profile was validated with simulations (triangular marks) and bench measurements (round marks), which depict a good overlap of the force profile and magnitudes.

As follows from the graph depicted in figure 12, the first overall magnetic force is a linear combination of the first transceiver-magnetic elements 5, 5a and first holding-magnetic elements 6, 6a and the second transceiver-magnetic elements 7, 7a and second holding-magnetic elements 8, 8a. In particular, figure 12 shows the inverse-square behavior of the first transceiver-magnetic elements 5, 5a and first holding-magnetic elements 6, 6a, or horizontal magnets, with a high attraction force at small distances along the connection axis C and quickly decaying for higher distances (triangular markers). Further, the second transceiver-magnetic elements 7, 7a and second holding-magnetic elements 8, 8a, or lateral magnetic elements, exert variable forces depending on the current position of the transceiver device 4 within the holding device 3 (round markers). They predominantly generate a downward force along the downward direction, i.e. towards the holding device 3 along the connection direction C, except when the transceiver device 4 is nearly completely inserted into the holding device 3 with a distance between the first transceiver-magnetic elements 5, 5a and the first holding-magnetic elements 6, 6a of below 2mm and with respect to the connection direction C. The overall magnetic force (cross markers) applied onto the probed body tissue 2 results from the linear combination of the downward forces, which are spatially separated far enough from each other to not influence each other. The resulting curve shows a more linearized behavior with a lowered overall force at distances shorter than 2 mm with respect to the connection direction C and an increased overall force at distances longer than 2 mm.

As further follows from the graph depicted in figure 15, different combinations of second magnetic elements are conceivable, such as using only one second magnetic-holding element 8, 8a or the proposed combination of a smaller 81 and larger 81a magnet from this invention. If only one magnet were to be used, there would be one distinct magnetic bump introduced pulling into the connection direction C for each magnet. Thus, it would either allow to generate a linearized force profile for distances <2 mm without attraction forces for distances >6mm, or vice versa it would provide a magnetic edge preventing the transceiver device 4 to disconnect easily while having excess forces for short distances below 2 mm. Thus, the combination of a short and long magnet generates a force field that combines the two intended functions of the single magnets.

For illustrative purposes, some example specifications of the connection mechanism provided by the transceiver device 4 and the holding device 3 and their overall magnetic force are given herein below.

### Example 1:

Force for a distance between the transceiver device 4 and the holding device 3 with respect to the connection direction C of 0-2 mm: This situation typically applies to body tissue in the form of head regions with long and dense hair. According to the state of the art and in line with our own experience, a pressure of 0.5-0.9 kg/cm2 is preferably achieved. With a transceiver area of 0.141 cm2 being provided at the end of the two contact elements 10 that each have a diameter of 3 mm and a gravity force of 9.81 m/s2, a possible force requirement of the transceiver assembly of 0.69-1.21 N results.

In such a case, the connecting element 10 of the transceiver device 4 protrudes maximally from the bottom side 19 of the transceiver device 4 as depicted in figure 11, corresponding to a minimal distance between the transceiver device 4 and the holding device 3, enabling the accommodation with a thick layer of hair or a loose cap.

### Example 2:

Force for a distance between the transceiver device 4 and the holding device 3 with respect to the connection direction C of 4-6 mm: This situation typically applies to body tissue in the form of bald head regions or any measurement on other body locations (e.g., arm, finger). For this configuration, a pressure range of 0.2-0.5 kg/cm² corresponds to a force requirement of 0.27-0.69 N for the transceiver assembly 1 according to the invention. The aim is to stay close to the lower threshold of this range to maximize comfort.

In such a case, the connecting element 10 of the transceiver device 4 protrudes minimally from the bottom side 19 of the transceiver device 4 as depicted in figure 10, corresponding to a larger distance between the transceiver device 4 and the holding device 3 of 4-6 mm, enabling measurements with bald body regions.

Further specifications:
Weight: A total weight of the transceiver assembly 1 according to the invention is preferably below 800 grams, more preferably below 600 grams, which corresponds to the weight of a ski helmet. For example, an exemplary transceiver assembly 1 according to the invention can comprise 50 transceiver devices 4, 4a, ... of 8 g, which results in a maximum overall weight of <200 g (i.e., <4 g for an individual holding device) for the connection mechanism being provided by the transceiver devices 4, 4a, ... and the holding devices 3, 3a, ... and the attaching device 12 such as a cap.

Attached state for bald head regions, wherein a stroke length, i.e. a maximal distance the tip of the contact element 10 can protrude from the lower edge of the holding device 27, of the transceiver device 4 with respect to the holding device 4 is 6 millimeters: In this state, the magnet-to-magnet distance between the first transceiver-magnetic elements 5, 5a and the first holding-magnetic elements 6, 6a, i.e. the bottom magnetic elements, in the transceiver device 4 and the holding device 3 is between 4.5 and 6.5 millimeter, which means that their attraction is low. To increase the pressure on the body tissue 2 such as the skin, the second transceiver-magnetic elements 7, 7a and the second holding-magnetic elements 8, 8a, i.e. the lateral magnetic elements, additionally generate a downward force toward the holding device 3. The two larger sub-magnetic elements 81a of the second holding-magnetic elements 8, 8a arranged in the side wall 24 of the holding device 3, generate a magnetic "edge" and prevent the transceiver device 4 from falling out of the holding device 3. That is, say magnetic "edge" can be seen as taking over the function of a mechanical stop that prevents a too easy disconnection of the transceiver device. It should be noted that such a magnetic "edge" can be provided in alternative manners. For instance, the magnetic "edge" could be generated from separate second-magnetic elements that are arranged with opposite polarities and that exert a repulsive force in addition to the existing setup. This would allow to create an "edge" that needs to be overcome once inserting the magnets.

Attached state for head regions with hair, wherein a stroke length of the transceiver device 4 with respect to the holding device 3 is between 0 to 2 millimeter: In this state, the magnet-to-magnet distance between the first transceiver-magnetic elements 5, 5a and the first holding-magnetic elements 6, 6a, i.e. the bottom magnetic elements, in the transceiver device 4 and the holding device 3 is between 0.5 to 2.5 millimeter. When the distance between holding device 3 and transceiver device 4 are minimal between each other, the intermagnet-distance corresponds to 0.5 millimeter, which means that their attraction is highest. To reduce the pressure on the body tissue such as the skin, the second transceiver-magnetic elements 7, 7a and the second holding-magnetic elements 8, 8a, i.e. the lateral magnetic elements, additionally generate an upward force away from the holding device 3 along the disconnection direction D.

Although the figures depict an exemplary transceiver assembly 1 that comprises two first and two second transceiver-magnetic elements 5, 5a; 7, 7a as well as to first and two second holding-magnetic elements 6, 6a; 8, 8a or a particular arrangement and design, many other number of magnetic elements, arrangements and designs are conceivable.

For example, a size or thickness of the magnetic elements 5, 5a; 6, 6a; 7, 7a; 8, 8a could be adjusted in order to change a force applied to the body tissue 2, particularly when the contacting element 10 protrudes significantly from the holding device 3. Instead of using two first transceiver-magnetic elements 5, 5a in the form of two round magnets, any number of magnetic elements that generate an attractive force could be employed. Additionally, a ring-shaped magnetic element could be combined with round magnetic elements in the transceiver device 4. This allows to design transceiver devices 4 with different geometries, and to employ smaller magnetic elements while exerting the same force onto the body tissue 2. Moreover, adjusting a thickness of the magnetic elements 5, 5a; 6, 6a; 7, 7a; 8, 8a allows for changes in the slope of the curve at specific regions, such as flattening the essentially exponential curve associated with the magnetic force over the distance between the holding device 3 and the transceiver device 4. The thickness can be altered within the transceiver device 4 or the holding device 3. Varying the thickness of the magnetic elements 5, 5a; 6, 6a; 7, 7a; 8, 8a enables influencing the inverse-square behavior of the magnetic force and reducing the size of the holding device 3 or transceiver device 4 by using thinner magnetic elements 5, 5a; 6, 6a; 7, 7a; 8, 8a. Modifying a size of the magnetic elements 5, 5a; 6, 6a; 7, 7a; 8, 8a in the z-direction or along the connection direction C (change of height of the magnetic elements 5, 5a; 6, 6a; 7, 7a; 8, 8a) permits adaptation of the location of the minimal force and the magnetic "edge" . In the x-direction or along the transverse direction T (change of width of the magnetic elements 5, 5a; 6, 6a; 7, 7a; 8, 8a), varying a size of the magnetic elements 5, 5a; 6, 6a; 7, 7a; 8, 8a alters the force applied to the body tissue 2, having the same effect as increasing the thickness of the magnetic elements 5, 5a; 6, 6a; 7, 7a; 8, 8a. Changing a position of the magnetic elements 5, 5a; 6, 6a; 7, 7a; 8, 8a, such as shifting the second transceiver-magnetic elements 7, 7a or the second holding-magnetic elements 8, 8a up or down, or such as titling of magnetic elements 5, 5a; 6, 6a; 7, 7a can impact the counteracting force of these magnetic elements 7, 7a; 8, 8a on the forces of the body tissue 2. For example, shifting these magnetic elements 7, 7a; 8, 8a upward results in a stronger reduction of the essentially exponential behavior, while shifting them downward could enhance it.

Hence, the transceiver assembly 1 according to the invention provides a novel attachment system that enhances usability and signal quality when performing biosignal measurements, for instance. Among its advantages are:
Compact size and simplicity: Minimizing obtrusiveness and facilitating manufacturing;
Ease of application: Enabling effortless and flexible transceiver device attachment to desired locations;
Comfort: Maximizing user comfort for prolonged and satisfactory usage;
Firm Contact with body tissue 2: Allowing adjustable pressure to ensure firm contact with the body tissue 2 regardless of characteristics of the body tissue 2 such as hair characteristics.

Moreover, the usability of most biosignal-sensing systems can be significantly improved by integrating the transceiver assembly 1 according to the invention. In particular, the transceiver assembly 1 can easily be integrated into existing headwear solutions such as caps or arm-like structures as well as any type of band around the trunk or limbs, and improve these systems' signal quality and ease of use.

The benefits of the magnet-based structure are:
Ease-of-use: The suggested connection mechanism enables a plug-and-play approach for biosignal transceiver device placement without the need to attach a separate mechanical part (e.g., cover for each holding device 3) or use a special tool for placement. Thanks to our approach, the transceiver device generates the optimal pressure on the body tissue 2 without any preparation or prior knowledge about the donning system.

Time-to-don: The time to don a biotransceiver with this connection mechanism is greatly reduced to comparable mechanisms. For bald regions, it only requires inserting it into the holding device, taking a matter of a few seconds for the entire head. For users with much hair, the transceiver devices must be rotated to probe through the hair, but the donning time is still significantly smaller compared to other solutions, and no special equipment is needed. Time-to-don is a crucial factor for time-sensitive applications, which is typically the case for all clinical or in-home applications.

Comfort: Thanks to an optimal force profile acting on the body tissue 2, the risk of pressure pain is small. The pressure is comfortable, whether for a bald person with a firmly attached cap or a person with dense hair and a loosely attached cap. A sufficiently large stroke length and increasing force allow the handling of such opposing situations.

Signal quality: Thanks to always having the right amount of pressure on the body tissue 2, the signal quality is optimized. A firm contact guarantees that no gap between the transceiver device and the body tissue 2 exists. At the same time, the connection mechanism prevents excess force that could lead to pain-induced physiological signals.

Robustness: With the ongoing transfer of brain imaging to clinical applications and freely moving subjects in research, it is crucial to have a solution that holds firmly enough on the skin to prevent motion artifacts. Since the magnetic elements are closely located to the body tissue 2 compared to a spring solution of the prior art, the weight of the connection mechanism is close to the head and induces only a minimal moment arm during movements. This improves the robustness of the transceiver assembly 1 compared to other solutions. Our solution also holds significant advantages compared to arm-like structures or fully integrated caps, as it can better compensate for movement-induced disturbances as long as the attachement assembly is placed firmly enough.

Another advantage is a significant gain in usability by reducing setup time and facilitating the handling of biosignal measurement systems. This enhances the user experience and addresses one of the main challenges in current wearable brain imaging systems. Additionally, it greatly benefits signal quality by providing a firm and comfortable contact with the skin and coping well with hair and motion artifacts.

**LIST OF REFERENCE SIGNS**

| | | | |
|---|---|---|---|
| 1 | transceiver assembly | 22, 22a | recess |
| 2 | body tissue | 23 | lid |
| 3, 3a, | holding device | 24 | side wall |
| 4, 4a, | transceiver device | 25 | top side |
| 5, 5a, | first transceiver-magnetic | 26 | bottom side |
| | element | 27 | bottom wall |
| 6, 6a, | first holding-magnetic | 28, 28a, | pins |
| | element | 29, 29a, | apertures |
| 7, 7a, | second transceiver-magnetic | 30, 30a, | recess |
| | element | 31, 31a, | recess |
| 8, 8a, | second holding-magnetic | 32, 32a | rotational stop element |
| | element | 33 | hair |
| 81, 81a, | sub-magnetic element | 34 | transmitter device |
| 9 | insertion opening | 35, 35a | receiver device |
| 10, 10a, | contacting element | 36 | analysing device |
| 11 | aperture | 37 | battery |
| 12 | attaching device | 38, 38a | light transmitting device |
| 13, 13a, ... | recess | | |
| 14 | holding-housing | C | connection direction |
| 15 | transceiver-housing | D | disconnection direction |
| 16 | side wall | T | transverse direction |
| 17 | opening | R | rotation axis |
| 18 | top side | pt1 | first transceiver plane |
| 19 | bottom side | pt2 | second transceiver plane |
| 20 | bottom wall | ph1 | first holding plane |
| 21, 21a | recess | ph2 | second holding plane |

## Claims

1. A transceiver assembly (1) for receiving signals of a body tissue (2) and/or transmitting signals to a body tissue (2) comprising:
- at least one holding device (3); and
- at least one transceiver device (4);
wherein the transceiver device (4) is configured to receive a signal from a body tissue (2) and/or to transmit a signal to a body tissue (2),
wherein the transceiver device (4) is connectable to the holding device (3) along a connection direction (C), and
wherein the transceiver device (4) comprises at least one first transceiver-magnetic element (5) and the holding device (3) comprises at least one first holding-magnetic element (6), the first transceiver-magnetic element (5) and the first holding-magnetic element (6) being arranged after one another with respect to the connection direction (C) and being configured to exert a magnetic force to one another,
**characterized in that** the transceiver device (4) comprises at least one second transceiver-magnetic element (7) and the holding device (3) comprises at least one second holding-magnetic element (8),
wherein the second transceiver-magnetic element (7) and the second holding-magnetic element (8) are arranged after one another with respect to a transverse direction (T) running perpendicularly to the connection direction (C) and are configured to exert a magnetic force to one another, and
wherein the transceiver device (4) is connectable to the holding device (3) via an overall magnetic force exerted by the first and second transceiver-magnetic elements (5; 7) and by the first and second holding-magnetic elements (6; 8).

2. The transceiver assembly (1) according to claim 1, wherein the first transceiver-magnetic element (5) and the first holding-magnetic element (6) are configured to exert an attractive magnetic force to one another, and/or
wherein the second transceiver-magnetic element (7) and the second holding-magnetic element (8) are configured to exert an attractive and/or repulsive magnetic force to one another.

3. The transceiver assembly (1) according to any one of the preceding claims, wherein the transceiver assembly (1) is configured to provide a variable pressure to the body tissue (2) that varies with respect to a position of the transceiver device (4) in relation to the holding device (3) and with respect to the connection direction (C).

4. The transceiver assembly (1) according to any one of the preceding claims, wherein the transceiver device (4) is movably connectable to the holding device (3) with respect to the connection direction (C) via the overall magnetic force.

5. The transceiver assembly (1) according to any one of the preceding claims, wherein the first and second transceiver-magnetic elements (5; 7) and the first and second holding-magnetic elements (6; 8) are arranged such, that their overall magnetic force is a linear combination of i) the magnetic force exerted between the first transceiver-magnetic element (5) and the first holding-magnetic element (6) and ii) the magnetic force exerted between the second transceiver-magnetic element (7) and the second holding-magnetic element (8).

6. The transceiver assembly (1) according to any one of the preceding claims, wherein the first transceiver-magnetic element (5) and the first holding-magnetic element (6) are arranged such that their exerted magnetic force is at least one of:
- attracting the transceiver device (4) towards the holding device (3) along the connection direction (C); or
- increasing with decreasing distance between the first transceiver-magnetic element (5) and the first holding-magnetic element (6) and with respect to the connection direction (C) in an essentially exponential manner.

7. The transceiver assembly (1) according to any one of the preceding claims, wherein the second transceiver-magnetic element (7) and the second holding-magnetic element (8) are arranged such that, depending on a position of the transceiver device (4) with respect to the holding device (3) and with respect to the connection direction (C), their magnetic force is attracting the transceiver device (4) towards the holding device (3) along the connection direction (C) or repelling the transceiver device (4) away from the holding device (3) along a disconnection direction (D) extending opposite to the connection direction (C).

8. The transceiver assembly (1) according to any one of the preceding claims, wherein the first transceiver-magnetic element (5) and the first holding-magnetic element (6) are arranged at least partially and preferably entirely overlapping with one another and with respect to the connection direction (C), and/or
wherein the second transceiver-magnetic element (7) and the second holding-magnetic element (8) are arranged at least partially overlapping with one another and with respect to the transverse direction (T).

9. The transceiver assembly (1) according to any one of the preceding claims, wherein at least one of:
- the first transceiver-magnetic element (5) extends within a first transceiver plane (pt1) and the second transceiver-magnetic element (7) extends within a second transceiver plane (pt2), the first transceiver plane (pt1) and the second transceiver plane (pt2) running at an angle and preferably perpendicularly to one another,
- the first holding-magnetic element (6) extends within a first holding plane (ph1) and the second holding-magnetic element (8) extends within a second holding plane (ph2), the first holding plane (ph1) and the second holding plane (ph2) running at an angle and preferably perpendicularly to one another, and
- the first transceiver plane (pt1) and the first holding plane (ph1) run parallel to one another and/or the second transceiver plane (pt2) and the second holding plane (ph2) run parallel to one another.

10. The transceiver assembly (1) according to any one of the preceding claims, wherein the holding device (3) comprises an insertion opening (9), and
wherein the transceiver device (4) is at least partially receivable in said insertion opening (9), and/or
wherein the transceiver device (4) is movably held within the insertion opening (9) via the overall magnetic force and preferably movable with respect to the connection direction (C).

11. The transceiver assembly (1) according to any one of the preceding claims, wherein the transceiver device (4) is rotatably connectable to the holding device (3) and is preferably rotatable about a rotation axis (R) running parallel to the connection direction (C).

12. The transceiver assembly (1) according to any one of the preceding claims, further comprising at least one contacting element (10) being configured to contact the body tissue (2), and
wherein the transceiver assembly (1) is configured to press the contacting element (10) against the body tissue (2) via the overall magnetic force, and/or
wherein the holding device (3) comprises at least one aperture (11) through which the contacting element (10) can at least partially protrude when contacting the body tissue (2).

13. The transceiver assembly (1) according to any one of the preceding claims, wherein the transceiver device (4) comprises at least one transmitter device (34) being configured to transmit the signal to the body tissue (2) and/or at least one receiver device (35, 35a) being configured to receive a signal from the body tissue (2), and
wherein the signal being transmitted to the body tissue (2) and/or being received from the body tissue (2) preferably is an optical signal and/or an electrical signal and/or an electromagnetic signal and/or an acoustic signal, and/or
wherein the transmitter device (34) and/or the receiver device (35, 35a) are preferably at least partially arranged in the contacting element (10).

14. The transceiver assembly (1) according to any one of the preceding claims, further comprising at least one attaching device (12) for attaching the holding device (3) to a body part of a human being or an animal, and
wherein the attaching device (12) preferably is a headwear such as a cap, a headband, or a headgear such as a virtual-reality headgear, or a bodywear being attachable to a trunk or a limb.

15. A method of manufacturing a transceiver assembly (1) for receiving signals of a body tissue (2) and/or transmitting signals to a body tissue (2), preferably a transceiver assembly (1) according to any one of the preceding claims, wherein the method comprises the steps of:
- Providing at least one holding device (3); and
- Providing at least one transceiver device (4);
wherein the transceiver device (4) is configured to receive a signal from a body tissue (2) and/or to transmit a signal to a body tissue (2),
wherein the transceiver device (4) is connectable to the holding device (3) along a connection direction (C), and
wherein the transceiver device (4) comprises at least one first transceiver-magnetic element and the holding device (3) comprises at least one first holding-magnetic element, the first transceiver-magnetic element and the first holding-magnetic element being arranged after one another with respect to the connection direction (C) and being configured to exert a magnetic force to one another,
**characterized in that** the transceiver device (4) comprises at least one second transceiver-magnetic element and the holding device (3) comprises at least one second holding-magnetic element,
wherein the second transceiver-magnetic element and the second holding-magnetic element are arranged after one another with respect to a transverse direction running perpendicularly to the connection direction (C) and are configured to exert a magnetic force to one another, and
wherein the transceiver device (4) is connectable to the holding device (3) via an overall magnetic force exerted by the first and second transceiver-magnetic elements and by the first and second holding-magnetic elements.
